# EUROPEAN PATENT SPECIFICATION

(11) **EP 3 441 097 B1**
(45) Date of publication and mention of the grant of the patent: **30.09.2020**
(21) Application number: 17185224.7
(22) Date of filing: 08.08.2017
(51) Int. Cl.: A61M 11/06

(54) **NEBULIZER FOR AEROSOLIZING A LIQUID WITH A DETACHABLE COVER INCLUDING A VALVE SYSTEM**
VERNEBLER ZUR AEROSOLVERSPRÜHUNG EINER FLÜSSIGKEIT MIT ABNEHMBAREM DECKEL MIT EINEM VENTILSYSTEM
ATOMISEUR POUR PULVÉRISER UN LIQUIDE AYANT UN COUVERCLE AMOVIBLE COMPRENANT UN SYSTÈME DE SOUPAPE

(43) Date of publication of application: 13.02.2019
(73) Proprietor: Air Liquide Medical Systems S.r.l., 20148 Milano (IT)
(72) Inventor: ALBERICI, Luca, 25030 RONCADELLE (BS) (IT); BUGATTI, Ottorino, 25068 Sarezzo (Bs) (IT); SANDONI, Giuseppe, 25124 BRESCIA (IT)
(74) Representative: Pittis, Olivier

(56) References cited:
- EP-A1- 0 626 180
- WO-A1-03/053500
- DE-C1- 19 902 847
- GB-A- 2 347 870
- GB-A- 2 358 356
- US-A1- 2011 253 134

## Description

The present invention concerns a valve system arranged on the detachable cover of a nebulizer useable in aerosoltherapy.

Nebulizing devices, commonly called nebulizers, are devices useable for delivering an inhalation therapy, i.e. an aerosoltherapy, to a patient in need thereof.

Nebulizers are used in combination with a source of gas, typically compressed air, for nebulizing a drug solution thereby obtaining a mist containing droplets having suitable sizes allowing them reaching the lower airways of the patient that has to inhale said mist in the course of his/her aerosoltherapy.

So far, different architectures of nebulizers have been proposed.

However, most of the current nebulizers are not suitable because they generally comprise a permanent opening for venting the CO₂-containing gases exhaled by the patient during the expiration phases.

The main drawback of such a permanent opening is that the drug-containing mist can also escape through said opening, thereby wasting a significant amount of drug in the environment, i.e. in the surrounding atmosphere.

Nebulising devices are disclosed for example in US2011/0253134, DE19902847C1, GB2358356A, EP0626180A1, wherein document EP0626180A1 discloses a device having features as in the preamble of the appended claim 1.

A goal of the present invention is to propose an improved nebulizer that limits the waste of drug in the surrounding atmosphere.

A solution according to the present invention is a nebulizer as defined in the appended independent claim 1.

In relation to the present invention :
- an "aerosol" is a mist containing droplets of liquid, for instance a liquid drug, and a gas, such as air,
- a "gas" is formed of a unique compound (i.e. a pure gas) or of several compounds (i.e. gas mixture) that is/are in gaseous form.
- a "liquid drug" or a "drug-containing solution" is a solution that comprises one or several liquid compounds and optionally one or several solid compounds dissolved, suspended or similarly incorporated in said one or several liquid compounds.

Depending on the embodiment, a nebulizer according to the present invention can comprise one or several of the following additional features:
- the one-way inspiration valve is configured for allowing gas, such as ambient air, to enter into the nebulization chamber of the nebulization body, during the inspiration phases of the patient, but for preventing any exit/escape of gas through said one-way inspiration valve during the expiration phases of the patient.
- the one-way expiration valve is configured for allowing gas, especially CO₂-containing gas, exiting the nebulization chamber during the expiration phases of the patient, but for preventing any entry of gas through said one-way expiration valve during the inspiration phases of the patient.
- the nebulizer further comprises a respiratory interface in fluid communication with the nebulization chamber.
- the respiratory interface comprises a mouthpiece, a mouth mask or a facial mask.
- the one-way expiration valve is located between the one-way inspiration valve and the respiratory interface, when the cover is integrally fixed to the nebulizer body.
- the reservoir comprises a liquid tank for receiving a liquid to be aerosolized.
- the liquid tank has a cup-like shape.
- the aerosol-generating system comprises a nozzle element comprising an orifice and a deflector element facing the orifice of the nozzle element.
- the aerosol-generating system comprises a nozzle element for accelerating the air delivered by a source of compressed air, thereby creating a Venturi or suction effect pulling some liquid solution out of the liquid tank.
- the aerosol-generating system comprises deflector element for atomizing the liquid thereby creating a mist containing liquid droplets.
- the nebulizer body further comprises an axially-arranged inner conduit comprising a lower end projecting into the liquid tank of the reservoir.
- the lower end of the inner conduit terminates close to the bottom of the liquid tank.
- the reservoir further comprises an inner conduit projecting axially and upwardly into the liquid tank.
- the axially-arranged inner conduit of the nebulizer body is arranged as a sleeve around a downstream end of the inner conduit of the reservoir.
- the axially-arranged inner conduit of the nebulizer body is spaced from the downstream end of the inner conduit of the reservoir by a spacing.
- the nozzle element and the deflector element of the aerosol-generating system are arranged into the lumen of the inner conduit of the nebulizer body.
- the inspiration valve of the cover is facing and in fluid communication with the air inlet located at the upper end of the axially-arranged inner conduit.
- the nebulization chamber is located above the liquid tank.
- the cover comprises a grip portion.
- the cover is detachably fixed to the nebulizer body.
- the cover is press-fitted, snap-fitted or similarly detachably affixable to the nebulizer body.
- the cover comprises a grip portion, such as a textured area, preferably arranged on its outer wall, that allows the user to easily decouple the cover from the nebulizer body.
- the one-way expiration valve is located between the one-way inspiration valve and the respiratory interface, when the cover is integrally fixed to the nebulizer body.
- the respiratory interface comprises a mouthpiece, a mouth mask or a facial mask.
- the nebulizer body, the reservoir and/or the cover are made of polymer, such as PP, PC, ABS, PA, PSU or any other suitable plastic material.
- the respiratory interface comprises an inner passage.
- the inner passage of the respiratory interface is in fluid communication with the nebulization chamber.
- the respiratory interface comprises a proximal end connected to the nebulizer body and a distal end that is free.
- the distal end of the respiratory interface comprises the exit orifice of the inner passage.

A preferred embodiment of a nebulizer according to the present invention is shown in the enclosed Figures, among which:
- Figure 1 represents an exploded view of an embodiment of a nebulizer according to the present invention,
- Figure 2 is an enlarged view of the cover of Figure 1,
- Figure 3 is a longitudinal sectional view of the nebulizer of Figure 1,
- Figure 4 is an elevation view of the reservoir of the nebulizer of Figure 1,
- Figures 5a-5c illustrate the assembling of the reservoir to the nebulizer body of the nebulizer of Figure 1,
- Figures 6a-6c illustrate the assembling of the cover to the nebulizer body of the assembly of Figure 5c, and
- Figures 7a-7b illustrate the connecting of a respiratory interface, e.g. a mouthpiece, to the nebulizer body of the assembly of Figures 6c.

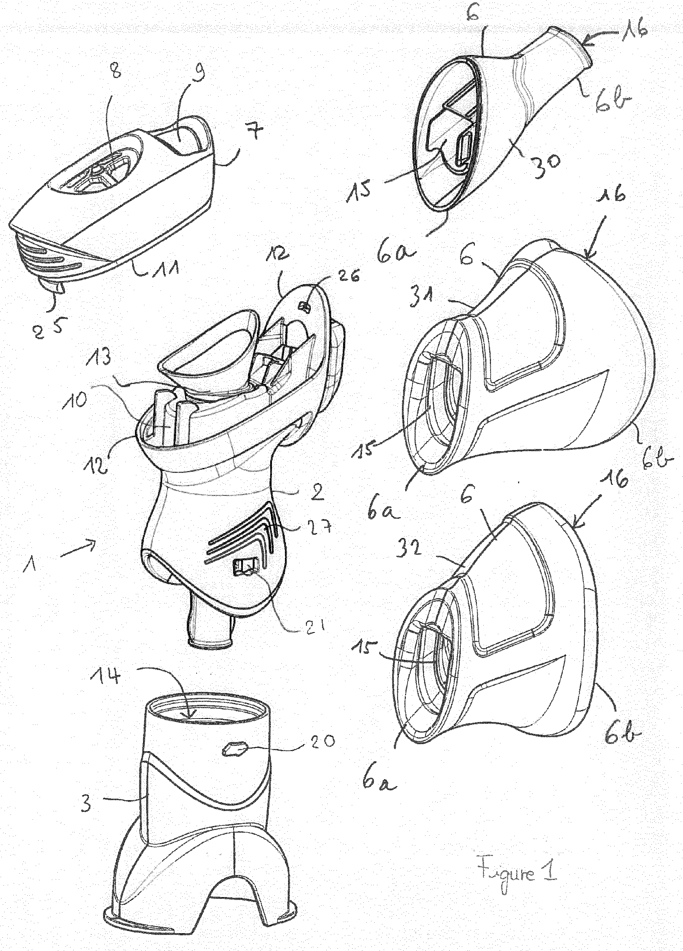

Figure 1 represents an exploded view an embodiment of a nebulizer 1 according to the present invention designed for aerosolizing a liquid, such as a drug useable in aerosoltherapy, which comprises:
- a reservoir 3 forming the lower part of the nebulizer 1, and which is designed for receiving and containing a liquid to be aerosolized, in particular a drug-containing solution,
- a nebulizer body 2 comprising an aerosol-generating system 4, 23, 24 for generating an aerosol, i.e. a mist, and a nebulization chamber 5 for containing the aerosol, i.e. the mist, generated by the aerosol-generating system 4, 23, 24,
- a cover 7 comprising a valve system 8, 9, and
- a respiratory interface 6 for delivering the drug-containing mist to a patient.

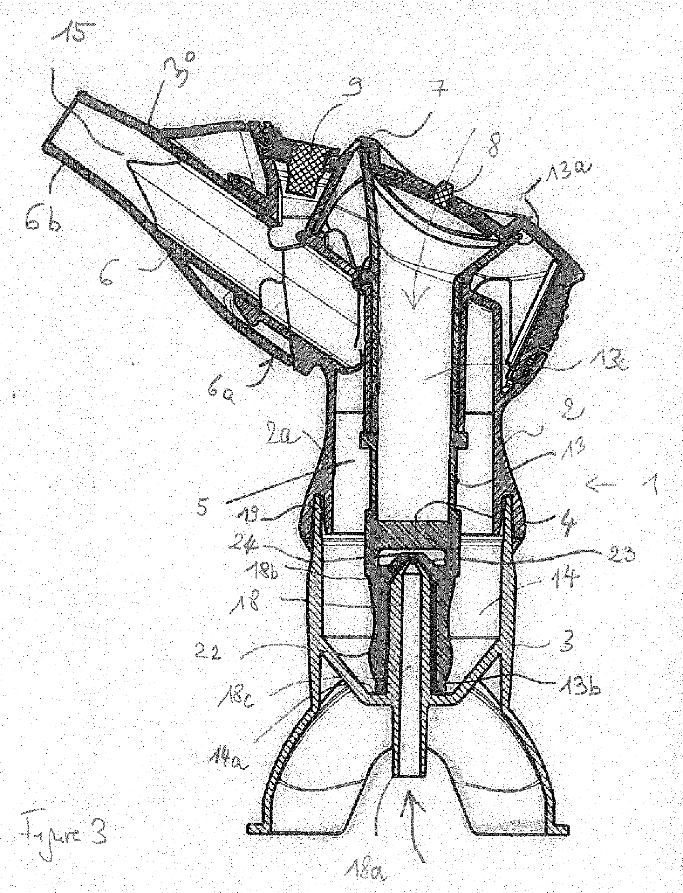

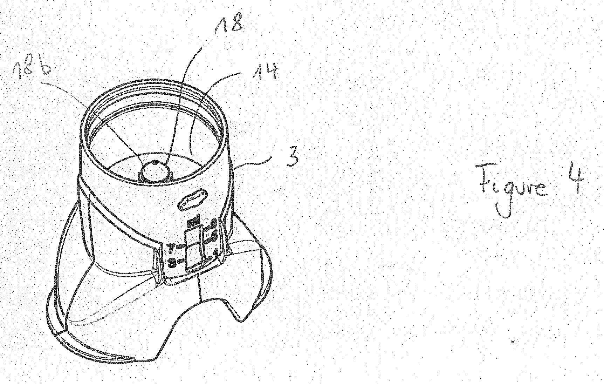

As shown in Figures 1, 3 and 4, the reservoir 3 comprises a liquid tank 14, also called liquid vessel or compartment, for receiving the liquid to be aerosolized, and further an inner conduit 18, as shown in Fig. 3, arranged so as to project axially and upwardly into the liquid tank 14 of the reservoir 3. Preferably, the tank 14 of the reservoir 3 has a "cup-like" shape.

Furthermore, the inner conduit 18 is arranged at the center of the liquid tank 14 of the reservoir 3. The role of the inner conduit 18 is to convey a pressurized gas delivered by a gas source (not shown), such as compressed air, fluidly connected to the upstream end 18a of the inner conduit 18. The gas flow travels into the lumen or inner passage 18c of the inner conduit 18 and is delivered by the downstream end 18b of the inner conduit 18 for creating the mist or aerosol as explained below.

Preferably, the reservoir 3 is detachably attached to the nebulizer body 2, for instance they are snap-fitted or threadedly-coupled together, or similarly adapted for repeated attachment/detachment.

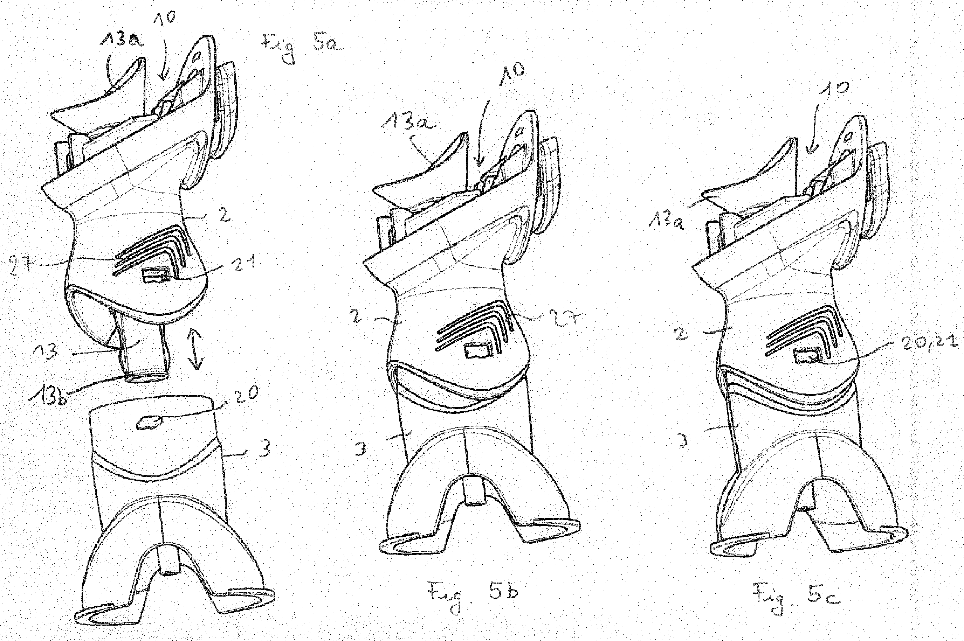

In the embodiment of Figure 1 and as shown in Figures 5a-5c, when the reservoir 3 is assembled by means of a snap-fit connection or similar (19 in Figure 3) to the nebulizer body 2, one (or several) picot or pin element(s) 20 arranged on the outer surface of the reservoir 3 fit(s) into, i.e. is (are) received into, one (or several) lodging(s) 21 arranged in the peripheral wall of the nebulizer body 2, i.e. bore(s) traversing the peripheral wall of the nebulizer body 2. This ensures a good positioning of the reservoir 3 with respect to the nebulizer body 2 as well as an improved fixation of those parts together, when they are snap-fitted or the like.

Further, the nebulizer body 2 forming the main part of the nebulizer 1 comprises a top opening 10 that is closed by an upper cover 7 forming the "top" or "roof" of the nebulizer body 2 as shown in Figure 1.

More precisely, the nebulizer body 2 comprises, as shown in Figure 3:
- an aerosol-generating system 4 for generating an aerosol, i.e. a mist,
- a nebulization chamber 5 for containing the aerosol, i.e. the mist, generated by the aerosol-generating system 4, and
- an axially-arranged inner conduit 13 comprising an air inlet at its upper end 13a, an air outlet 13b at its lower end 13b and an internal passage 13c arranged in-between for conveying ambient air from the air inlet towards the air outlet.

Preferably, at least a part of the nebulization chamber 5 is positioned between the peripheral wall 2a of the nebulizer body 2 and the axially-arranged inner conduit 13, and further located above the liquid tank 14.

As shown in Figure 3, when the reservoir 3 is assembled to the nebulizer body 2, the downstream end 18b of the inner conduit 18 is inserted into the internal passage 13c of the lower end 13b of the axially-arranged inner conduit 13, i.e. the downstream end 18b of the inner conduit 18 passes through the air outlet 13b of the the axially-arranged inner conduit 13 of the nebulizer body 2.

In other words, the lower end 13b of the axially-arranged inner conduit 13 of the nebulizer body 2 forms a sleeve around the downstream end 18b of the inner conduit 18 of the reservoir 3.

The lower end 13b of the axially-arranged inner conduit 13 of the nebulizer body 2 further projects into the liquid tank 14 of the reservoir 3 and terminates close to the bottom 14a of the liquid tank 14 as shown in Figure 3, preferably at a distance of less than about 2 mm.

In the aim of allowing a circulation of liquid between them, a spacing 22 is kept, on the one hand, between the extremity of the lower end 13b of the axially-arranged inner conduit 13 and the bottom 14a of the liquid tank 14 and, on the second hand, between the inner wall of the lower end 13b of the axially-arranged inner conduit 13 and the outer wall of the downstream end 18b of the inner conduit 18 of the reservoir 3.

Preferably, the distance between the inner wall of the lower end 13b of the axially-arranged inner conduit 13 and the outer wall of the downstream end 18b of the inner conduit 18 of the reservoir 3 is of less than about 1 mm.

Indeed, for generating the aerosol mist, the liquid contained into the tank 14 of the reservoir 3 should be able to circulate in said spacing 22 for reaching the aerosol-generating system 4, 23, 24, as explained below.

The aerosol-generating system 4, 23, 24 is arranged in the lumen 13c of the inner conduit 13 of the nebulizer body 2 and comprises, as shown in Figure 3 :
- a nozzle element 23 with a small orifice 24 forming a flow restriction, for instance a restriction orifice 24 having a diameter or dimensions of between about 1 mm to 5 mm. The orifice 24 can have a circular section, an ellipse section or any other suitable shape. Preferably, the nozzle element 23 is axially arranged into lumen 13c.
- and a deflector element 4 facing the small orifice 24 of the nozzle element 23, such as a little wall, blade, pin or similar that is arranged into lumen 13c, preferably radially-positioned into lumen 13c.

Such an aerosol-generating system 4, 23, 24 can generate the aerosol mist thanks to a Venturi effect. This principle is well-known in the art. In few words, pressurized air is conveyed and delivered by by the inner conduit 18 of the reservoir 3, at a high speed and a constant flowrate. This creates a suction effect that moves the liquid solution out of the tank 14. The liquid solution travels in the spacing 22, i.e. it is upwardly pulled by the suction effect, and is then delivered by the small orifice 24 of the nozzle element 23 toward the deflector element 4. When the liquid enters into contact with the deflector element 4, a mist of liquid droplets is created by the impact of the liquid on the deflector element 4. The smaller droplets create the desired aerosol mist that is recovered into the nebulization chamber 5, whereas the larger and/or heavier droplets return to the liquid tank 14. The aerosol mist can thereafter be inhaled by the patient via a respiratory interface 6.

Indeed, a respiratory interface 6, also called "patient interface", is fixed to the nebulizer body 2 and is in fluid communication with the nebulization chamber 5 so that the aerosol created in the nebulization chamber 5 can be delivered to a patient by said respiratory interface 6 as illustrated in Figure 3.

As shown in Figure 1, the respiratory interface 6 can be a mouthpiece 30, or a mouth or facial respiratory mask 31, 32, or similar. The choice of the most suitable interface 6 depends on the type of medication to be taken and on the type of patients i.e. the facial mask is suitable for non collaborative pediatric patients, while the mouthpiece and mouth mask are better for adults.

In the embodiment of Figures 1,3 and 7a-7b, the respiratory interface 6 is a mouthpiece 30 with an inner passage 15, that is designed so that the patient takes it into his/her mouth for inhaling the drug-containing mist. Actually, the mouthpiece 30 is traversed by an inner passage 15 that is in fluid communication with the nebulization chamber 5.

Generally speaking, the respiratory interface 6 comprises a proximal end 6a connected to the nebulizer body 2 and a distal end 6b that is free and which comprises the exit orifice 16 of the inner passage 15.

In the case of a mouthpiece 30, the distal end 6b is further designed so as to form a "beak-shape nozzle" that the patient takes in mouth, whereas in the case of a mouth or facial respiratory mask 31, 32, the distal end 6b is further designed so as to form an enclosure that is large enough for receiving at least a part of the patient's nose or nose and mouth, through the exit orifice 16.

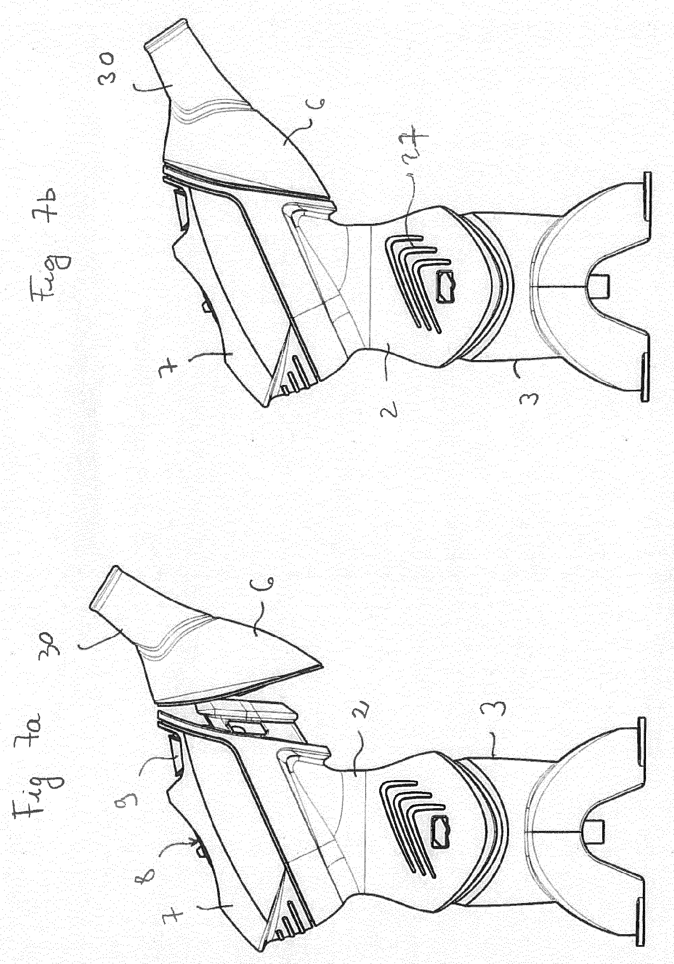

Figures 7a-7b show the attachment of a mouthpiece 30 to the nebulizer body 2 of the nebulizer 1 of Figure 1. However, attaching of a mouth or facial respiratory mask 31, 32 can be operated similarly.

Furthermore, the nebulizer 1 further comprises a detachable cover 7 that forms the "top" of the nebulizer 1 as forming the ceiling of the nebulizer body 2 as illustrated in Figures 1, 2 and 3.

More precisely, the cover 7 comprises a lower peripheral border 11 cooperating with an upper peripheral border 12 delimiting the top opening 10 of the nebulizer body 2.

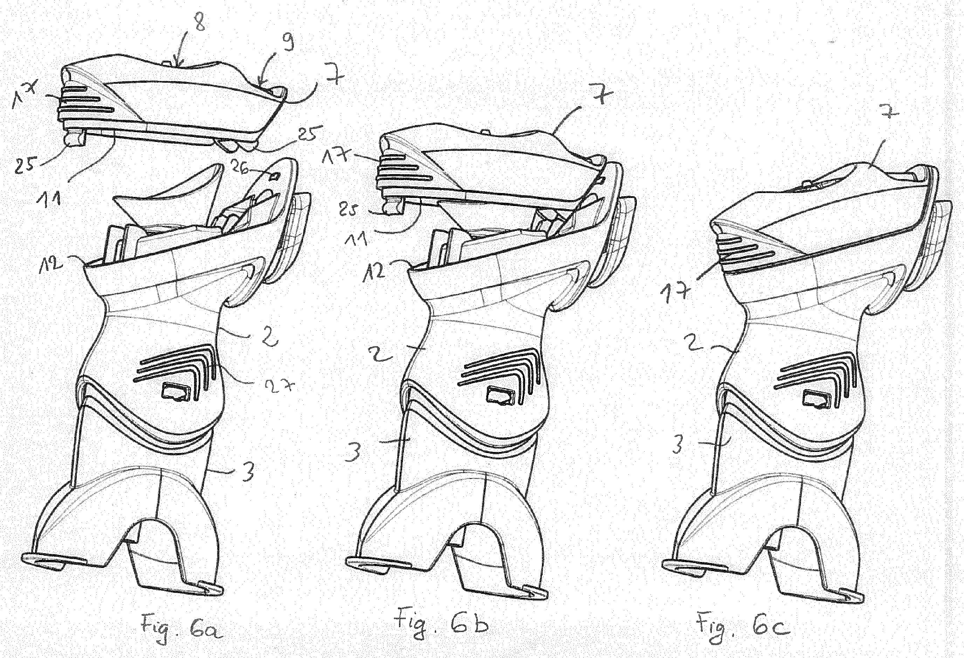

For maintaining the cover 7 detachably attached to the nebulizer body 2, the cover 7 can, for instance, be snap-fitted or similar to the nebulizer body 2 so that the lower peripheral border 11 of the cover 7 cooperates with the upper peripheral border 12 of the top opening 10 of the nebulizer body 2. As shown in Figures 1, 2 and 6a-6c, connecting structures 25, 26, such as pin(s), finger(s), claw(s), clamp(s) 25 or the like and corresponding lodging(s) 26, groove(s) or the like, are arranged on the cover 7 and/or the nebulizer body 2 and cooperate together for allowing a tight and integral fixing of the cover 7 to the nebulizer body 2.

Preferably, the top cover 7 further comprises a grip portion 17 arranged in its outer wall that allows the user, e.g. the patient, to easily remove the cover 7 when attached to the nebulizer body 2, for instance for cleaning the nebulization chamber 5. The grip portion 17 can comprise a textured area, i.e. a not-smooth surface, allowing a good grip to the user, e.g. a textured area comprising "ridges" and "valleys".

Furthermore, similar grip portions 27 are also arranged in the peripheral wall 2a of the nebulizer body 2 for improving the hand grip of the nebulizer 1 by the patient.

All the components of the nebulizer 1, i.e. cover 7, nebulizer body 2..., are preferably made of plastic material(s), such as PP, PC, ABS, PA, PSU or similar materials, while the masks are made of a soft elastomeric material, such as silicone or the like.

Further, according to the present invention, the cover 7 comprises a valve system 8, 9 comprising a pair of valves, i.e.
- a one-way inspiration valve 8 allowing ambient air entering into the nebulization chamber 5 via the axially-arranged inner conduit 13 of the nebulizer body 2, during the inspiration phases of the patient, and
- a one-way expiration valve 9 allowing gas exiting the nebulization chamber 5, during the expiration phases of the patient, especially expired gases that contains CO₂.

Preferably, the one-way expiration valve 9 is located between the one-way inspiration valve 8 and the respiratory interface 6, when the cover 7 is integrally fixed to the nebulizer body 2.

In other words, the inspiration valve 8 is located on the cover 7 facing and in fluid communication with the air inlet located at the upper end 13a of the axially-arranged inner conduit 13, and opens only during inspiration phases of the patient by action of the negative pressure generated by the patient's inspiration, i.e. during inspiration phases.

The inspiration valve 8 provides an additional flow of ambient air that enters into the nebulization chamber 5 thereby boosting the nebulization (due to a "double" Venturi effect) and significantly increasing the amount of aerosol mist formed into said nebulizing chamber 5, and further thereby increasing the nebulization rate of the liquid drug, i.e. the solution containing the medication.

In other words, the on-way inspiration valve 8 allows air to only travel from the outside to the inside of the nebulizer 1, i.e. to enter into the nebulization chamber 5 via the axially-arranged inner conduit 13.

Further, the expiration valve 9 is located next to the inspiration valve 8 on the detachable cover 7, but closer to the mouthpiece 6 than the inspiration valve 8.

Said expiration valve 9 opens by action of the CO₂-containing gases exhaled by the patient that create an overpressure (i.e. pressure > 1 bar) in the respiratory interface 6 and at the inlet of the nebulization chamber 5, while the patient is exhaling gases. Said overpressure allows gases to be vented from the nebulizer 1, through the expiration valve 9, when the patient exhales into the respiratory interface 6. In other words, the on-way expiration valve 9 allows said CO₂-containing gas to only travel from the inside to the outside of the nebulizer 1, i.e. to be vented to the atmosphere.

With a nebulizer 1 according to the present invention, the patient does not remove the respiratory interface 6, such as the mouthpiece 30 out of his/her mouth, before starting to expire CO₂-containing gases.

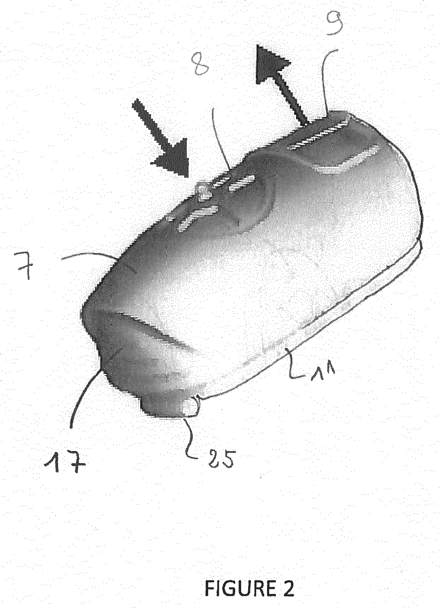

In other words, thanks to the nebulizer 1 according to the present invention, the patient can execute his/her aerosoltherapy by both inhaling and exhaling gases through the respiratory interface 6 and the cover 7 (see arrows in Fig. 2), the amount of aerosolized drug wasted in the environment is considerably reduced, compared with prior art nebulizers, as the exit of expired gases is controlled by the one-way expiration valve 9, i.e. there is no permanent opening for exhaled air.

Furthermore, it is advantageous to arranged both valves 8, 9 on the detachable cover 7, rather than on the respiratory interface 6 because it reduces the number of expiration valves that otherwise would have been necessary for using the nebulizer 1 with different kinds of respiratory interfaces 6, i.e. mouthpiece 30, mouth and facial masks 31, 32, each equipped with an expiration valve.

The nebulizer 1 of the present invention is usable in combination with a source of gas, such as compressed air, for nebulizing a drug-containing solution and thereby obtaining a mist containing droplets of medication having suitable sizes so as to be able to reach the lower airways of the patient that has to inhale said mist in the frame of his/her aerosoltherapy.

## Claims

1. Nebulizer (1) for aerosolizing a liquid comprising :
- a reservoir (3) for receiving a liquid to be aerosolized,
- a nebulizer body (2) comprising an aerosol-generating system (4; 23, 24) for generating an aerosol and a nebulization chamber (5) for containing the aerosol, said aerosol-generating system (4; 23, 24) comprising a nozzle element (23) comprising an orifice (24) and a deflector element (4) facing the orifice (24) of the nozzle element (23), ;
- a cover (7) forming the top of the nebulizer (1), the cover (7) being detachably fixed to the nebulizer body (2), and further comprising a one-way inspiration valve (8) allowing air entering into the nebulization chamber (5) ; wherein
- the reservoir (3) comprises a liquid tank (14) for receiving a liquid to be aerosolized, and an inner conduit (18) projecting axially and upwardly into the liquid tank (14),
- the nebulizer body (2) comprises an axially-arranged inner conduit (13) comprising a lower end (13b) projecting into the liquid tank (14) of the reservoir (3), the lower end (13b) of the inner conduit (13) terminating close to the bottom (14a) of the liquid tank (14), the axially-arranged inner conduit (13) being arranged as a sleeve around a downstream end (18b) of the inner conduit (18) of the reservoir (3),
- the nozzle element (23) and the deflector element (4) of the aerosol-generating system (4; 23, 24) are arranged into the lumen (13c) of the inner conduit (13) of the nebulizer body (2), and
- the inspiration valve (8) of the cover (7) is facing and in fluid communication with an air inlet located at an upper end (13a) of the axially-arranged inner conduit (13),
**characterized in that**
the cover further comprises a one-way expiration valve (9) allowing gas exiting the nebulization chamber (5).

2. Nebulizer according to Claim 1, **characterized in that** it further comprises a respiratory interface (6) in fluid communication with the nebulization chamber (5).

3. Nebulizer according to Claim 1, **characterized in that** the one-way expiration valve (9) is located between the one-way inspiration valve (8) and the respiratory interface (6), when the cover (7) is integrally fixed to the nebulizer body (2).

4. Nebulizer according to Claims 1 or 2, **characterized in that** the respiratory interface (6) comprises a mouthpiece (30), a mouth mask (31) or a facial mask (32).

5. Nebulizer according to Claim 1, **characterized in that** the axially-arranged inner conduit (13) of the nebulizer body (2) is spaced from the downstream end (18b) of the inner conduit (18) of the reservoir (3) by a spacing (22).

6. Nebulizer according to Claims 1 or 2, **characterized in that** the nebulization chamber (5) is located above the liquid tank (14).

7. Nebulizer according to Claims 1 or 3, **characterized in that** the cover (7) comprises a grip portion (17).

8. Nebulizer according to Claim 1, **characterized in that** at least one pin elements (20) arranged on an outer surface of the reservoir (3) fits into at least one lodging (21) arranged in a peripheral wall of the nebulizer body (2) thereby assembling the reservoir (3) to the nebulizer body (2) by means of a snap-fit connection (19).

9. Nebulizer according to Claims 1 or 8, **characterized in that** several pin elements (20) arranged on the outer surface of the reservoir (3) fit into several lodgings (21) arranged in the peripheral wall of the nebulizer body (2).

10. Nebulizer according to Claims 8 or 9, **characterized in that** said one or several lodgings (21) arranged in the peripheral wall of the nebulizer body (2) are one or several bores traversing the peripheral wall of the nebulizer body (2).

11. Nebulizer according to Claim 1, **characterized in that** the reservoir (3) is detachably attached to the nebulizer body (2).

12. Nebulizer according to Claim 1, characterized in that₋at least a part of the nebulization chamber (5) is positioned between the peripheral wall (2a) of the nebulizer body (2) and the axially-arranged inner conduit (13), and further located above the liquid tank (14).

13. Nebulizer according to Claim 1, **characterized in that** the lower end (13b) of the inner conduit (13) terminating at a distance of less than about 2 mm from the bottom (14a) of the liquid tank (14).

## Patentansprüche

1. Vernebler (1) zum Aerosolisieren einer Flüssigkeit, umfassend:
- ein Reservoir (3) zum Aufnehmen einer zu aerosolisierenden Flüssigkeit,
- einen Verneblerkörper (2), der ein aerosolerzeugendes System (4; 23, 24) zum Erzeugen eines Aerosols und eine Verneblungskammer (5) zum Enthalten des Aerosols umfasst, wobei das aerosolerzeugende System (4; 23, 24) ein Düsenelement (23) umfasst, das eine Öffnung (24) und ein zu der Öffnung (24) des Düsenelements (23) zeigendes Deflektorelement (4) umfasst,
- eine Abdeckung (7), die die Oberseite des Verneblers (1) bildet, wobei die Abdeckung (7) abnehmbar an dem Verneblerkörper (2) befestigt ist, und weiter ein Einatmungsrückschlagventil (8) umfasst, das erlaubt, dass Luft in die Verneblungskammer (5) eintritt; wobei
- das Reservoir (3) einen Flüssigkeitsbehälter (14) zum Aufnehmen einer zu aerosolisierenden Flüssigkeit und ein inneres Leitungsrohr (18), das axial und nach oben in den Flüssigkeitsbehälter (14) ragt, umfasst,
- der Verneblerkörper (2) ein axial angeordnetes inneres Leitungsrohr (13) umfasst, das ein unteres Ende (13b) umfasst, das in den Flüssigkeitsbehälter (14) des Reservoirs (3) ragt, wobei das untere Ende (13b) des inneren Leitungsrohrs (13) nahe dem Boden (14a) des Flüssigkeitsbehälters (14) endet, wobei das axial angeordnete innere Leitungsrohr (13) als eine Manschette um ein stromabwärtiges Ende (18b) des inneren Leitungsrohrs (18) des Reservoirs (3) angeordnet ist,
- das Düsenelement (23) und das Deflektorelement (4) des aerosolerzeugenden Systems (4; 23, 24) in dem Lumen (13c) des inneren Leitungsrohrs (13) des Verneblerkörpers (2) angeordnet sind, und
- das Einatmungsventil (8) der Abdeckung (7) zu einem an einem oberen Ende (13a) des axial angeordneten inneren Leitungsrohrs (13) befindlichen Lufteinlass zeigt und damit in Fluidkommunikation steht,
**dadurch gekennzeichnet, dass**
die Abdeckung weiter ein Ausatmungsrückschlagventil (9) umfasst, das erlaubt, dass Gas aus der Verneblungskammer (5) austritt.

2. Vernebler nach Anspruch 1, **dadurch gekennzeichnet, dass** er weiter eine Atemwegsschnittstelle (6) in Fluidkommunikation mit der Verneblungskammer (5) umfasst.

3. Vernebler nach Anspruch 1, **dadurch gekennzeichnet, dass** sich das Ausatmungsrückschlagventil (9) zwischen dem Einatmungsrückschlagventil (8) und der Atemwegschnittstelle (6) befindet, wenn die Abdeckung (7) einstückig an dem Verneblerkörper (2) befestigt ist.

4. Vernebler nach Anspruch 1 oder 2, **dadurch gekennzeichnet, dass** die Atemwegsschnittstelle (6) ein Mundstück (30), eine Mundmaske (31) oder eine Gesichtsmaske (32) umfasst.

5. Vernebler nach Anspruch 1, **dadurch gekennzeichnet, dass** das axial angeordnete innere Leitungsrohr (13) des Verneblerkörpers (2) von dem stromabwärtigen Ende (18b) des inneren Leitungsrohrs (18) des Reservoirs (3) durch einen Zwischenraum (22) beabstandet ist.

6. Vernebler nach Anspruch 1 oder 2, **dadurch gekennzeichnet, dass** sich die Verneblungskammer (5) über dem Flüssigkeitsbehälter (14) befindet.

7. Vernebler nach Anspruch 1 oder 3, **dadurch gekennzeichnet, dass** die Abdeckung (7) einen Greifabschnitt (17) umfasst.

8. Vernebler nach Anspruch 1, **dadurch gekennzeichnet, dass** mindestens ein Stiftelemente (20), angeordnet auf einer Außenfläche des Reservoirs (3), in mindestens eine Aufnahme (21), angeordnet in einer peripheren Wand des Verneblerkörpers (2), passt, wodurch das Reservoir (3) mittels eines Schnappverschlusses (19) mit dem Verneblerkörper (2) zusammengefügt wird.

9. Vernebler nach Anspruch 1 oder 8, **dadurch gekennzeichnet, dass** mehrere Stiftelemente (20), angeordnet auf der Außenfläche des Reservoirs (3), in verschiedene Aufnahmen (21), angeordnet in der peripheren Wand des Verneblerkörpers (2), passen.

10. Vernebler nach Anspruch 8 oder 9, **dadurch gekennzeichnet, dass** die eine oder die mehreren Aufnahmen (21), angeordnet in der peripheren Wand des Verneblerkörpers (2), eine oder mehrere Bohrungen sind, die durch die periphere Wand des Verneblerkörpers (2) verlaufen.

11. Vernebler nach Anspruch 1, **dadurch gekennzeichnet, dass** das Reservoir (3) abnehmbar an dem Verneblerkörper (2) angebracht ist.

12. Vernebler nach Anspruch 1, **dadurch gekennzeichnet, dass** mindestens ein Teil der Verneblungskammer (5) zwischen der peripheren Wand (2a) des Verneblerkörpers (2) und dem axial angeordneten inneren Leitungsrohr (13) positioniert ist und sich weiter über dem Flüssigkeitsbehälter (14) befindet.

13. Vernebler nach Anspruch 1, **dadurch gekennzeichnet, dass** das untere Ende (13b) des inneren Leitungsrohrs (13) in einem Abstand von weniger als etwa 2 mm vom Boden (14a) des Flüssigkeitsbehälters (14) endend.

## Revendications

1. Atomiseur (1) pour pulvériser un liquide comprenant :
- un réservoir (3) pour recevoir un liquide à pulvériser,
- un corps d'atomiseur (2) comprenant un système de génération d'aérosol (4 ; 23, 24) pour générer un aérosol et une chambre d'atomisation (5) pour contenir l'aérosol, ledit système de génération d'aérosol (4 ; 23, 24) comprenant un élément de buse (23) comprenant un orifice (24) et un élément de déflecteur (4) faisant face à l'orifice (24) de l'élément de buse (23),
- un couvercle (7) formant la partie supérieur de l'atomiseur (1), le couvercle (7) étant fixé de manière amovible au corps d'atomiseur (2), et comprenant en outre une valve d'inspiration unidirectionnelle (8) permettant à de l'air d'entrer dans la chambre d'atomisation (5) ; dans lequel
- le réservoir (3) comprend un réservoir de liquide (14) pour recevoir un liquide à pulvériser, et un conduit intérieur (18) faisant saillie axialement et vers le haut dans le réservoir de liquide (14),
- le corps d'atomiseur (2) comprend un conduit intérieur agencé axialement (13) comprenant une extrémité inférieure (13b) faisant saillie dans le réservoir de liquide (14) du réservoir (3), l'extrémité inférieure (13b) du conduit intérieur (13) se terminant près du fond (14a) du réservoir de liquide (14), le conduit intérieur agencé axialement (13) étant agencé comme un manchon autour d'une extrémité aval (18b) du conduit intérieur (18) du réservoir (3),
- l'élément de buse (23) et l'élément de déflecteur (4) du système de génération d'aérosol (4 ; 23, 24) sont agencés dans la lumière (13c) du conduit intérieur (13) du corps d'atomiseur (2), et
- la valve d'inspiration (8) du couvercle (7) fait face et est en communication fluidique avec une entrée d'air située au niveau d'une extrémité supérieure (13a) du conduit intérieur agencé axialement (13),
**caractérisé en ce que**
le couvercle comprend en outre une valve d'expiration unidirectionnelle (9) permettant à du gaz de sortir de la chambre d'atomisation (5).

2. Atomiseur selon la revendication 1, **caractérisé en ce qu'**il comprend en outre une interface respiratoire (6) en communication fluidique avec la chambre d'atomisation (5).

3. Atomiseur selon la revendication 1, **caractérisé en ce que** la valve d'expiration unidirectionnelle (9) est située entre la valve d'inspiration unidirectionnelle (8) et l'interface respiratoire (6), lorsque le couvercle (7) est intégralement fixé au corps d'atomiseur (2).

4. Atomiseur selon les revendications 1 ou 2, **caractérisé en ce que** l'interface respiratoire (6) comprend un embout buccal (30), un masque buccal (31) ou un masque facial (32).

5. Atomiseur selon la revendication 1, **caractérisé en ce que** le conduit intérieur agencé axialement (13) du corps d'atomiseur (2) est écarté de l'extrémité aval (18b) du conduit intérieur (18) du réservoir (3) par un écartement (22).

6. Atomiseur selon les revendications 1 ou 2, **caractérisé en ce que** la chambre d'atomisation (5) est située au-dessus du réservoir de liquide (14).

7. Atomiseur selon les revendications 1 ou 3, **caractérisé en ce que** le couvercle (7) comprend une portion de préhension (17).

8. Atomiseur selon la revendication 1, **caractérisé en ce qu'**au moins un éléments de goupille (20) agencé sur une surface extérieure du réservoir (3) s'ajuste dans au moins un logement (21) agencé dans une paroi périphérique du corps d'atomiseur (2) assemblant ainsi le réservoir (3) au corps d'atomiseur (2) au moyen d'un raccordement d'encliquetage (19).

9. Atomiseur selon les revendications 1 ou 8, **caractérisé en ce que** plusieurs éléments de goupille (20) agencés sur la surface extérieure du réservoir (3) s'ajustent dans plusieurs logements (21) agencés dans la paroi périphérique du corps d'atomiseur (2).

10. Atomiseur selon les revendications 8 ou 9, **caractérisé en ce que** lesdits un ou plusieurs logements (21) agencés dans la paroi périphérique du corps d'atomiseur (2) sont un ou plusieurs trous traversant la paroi périphérique du corps d'atomiseur (2).

11. Atomiseur selon la revendication 1, **caractérisé en ce que** le réservoir (3) est attaché de manière amovible au corps d'atomiseur (2).

12. Atomiseur selon la revendication 1, **caractérisé en ce qu'**au moins une partie de la chambre d'atomisation (5) est positionnée entre la paroi périphérique (2a) du corps d'atomiseur (2) et le conduit intérieur agencé axialement (13), et en outre située au-dessus du réservoir de liquide (14).

13. Atomiseur selon la revendication 1, **caractérisé en ce que** l'extrémité inférieure (13b) du conduit intérieur (13) se terminant à une distance inférieure à environ 2 mm du fond (14a) du réservoir de liquide (14).
